# EUROPEAN PATENT SPECIFICATION

(11) **EP 4 132 469 B1**
(45) Date of publication and mention of the grant of the patent: **29.11.2023**
(21) Application number: 21721184.6
(22) Date of filing: 01.04.2021
(51) Int. Cl.: A61K 9/107, A61K 31/436, A61K 31/675, A61K 47/24, A61P 25/00, A61P 35/00

(54) **ADMINISTRATION OF MTOR INHIBITORS INTO THE CENTRAL NERVOUS SYSTEM**
VERABREICHUNG VON MTOR-HEMMERN IN DAS ZENTRALE NERVENSYSTEM
ADMINISTRATION D'INHIBITEURS DE MTOR DANS LE SYSTÈME NERVEUX CENTRAL

(30) Priority: 06.04.2020 IT 202000007228
(43) Date of publication of application: 15.02.2023
(73) Proprietor: Dolcetta, Diego, 36100 Vicenza (IT)
(72) Inventor: GIOVAGNOLI, Stefano, 06124 Perugia (IT); MAGINI, Alessandro, 06125 Perugia (IT); DOLCETTA, Diego, 36100 Vicenza (IT)
(74) Representative: Delbarba, Andrea
(86) International application number: PCT/IB2021/052740
(87) International publication number: WO 2021/205297

(56) References cited:
- EP-B1- 2 846 770
- WO-A2-2006/110862
- TOMMASO CASSANO ET AL: "Early intrathecal infusion of everolimus restores cognitive function and mood in a murine model of Alzheimer's disease", EXPERIMENTAL NEUROLOGY, vol. 311, 20 September 2018 (2018-09-20), pages 88-105, XP055757663, AMSTERDAM, NL ISSN: 0014-4886, DOI: 10.1016/j.expneurol.2018.09.011

## Description

### FIELD OF THE INVENTION

The present invention relates to lipid vesicles containing at least one mTOR inhibitor for use in the treatment or prevention of a disease of the central nervous system, and compositions comprising the lipid vesicles for use in said treatment or prevention.

### STATE OF THE ART

The *mammalian target of rapamycin* (mTOR) is a serine/threonine kinase with the function of promoting cell growth, proliferation, protein synthesis and survival in response to the environment and cell requirements. This enzymatic complex is formed by two molecular subunits called mTOR Complex 1 (mTORC1) and mTOR Complex 2 (mTORC2), both formed by the association of several proteins.

Ample research in the last twenty years has demonstrated that mTOR influences growth, metabolism and cell aging in response to environmental and endocrine stimuli.

Rapamycin and the analogues thereof are known as mTOR inhibitors capable of blocking the activity mainly of mTORC1. Rapamycin derivatives (*rapalogs*) are molecules developed with the objective of improving the pharmacokinetic and toxicological profile of the parent. They share with the latter the same molecular *scaffold* but have substitutions in the hydroxyl group at the C40 position of the methoxycyclohexanol residue, adapted to modulate the physicochemical properties, biodistribution and metabolism thereof.

The effectiveness of mTOR inhibitors in the treatment of numerous pathologies affecting the central nervous system (CNS) has been amply demonstrated. Various clinical studies have been conducted to assess the benefit of mTOR inhibitors in the treatment of the tuberous sclerosis (TS), a genetic syndrome characterised by the occurrence of tumours in various organs and tissues: mTOR inhibitors are effective in inducing regression of the cerebellar astrocytomas typical of this condition. However, the pathology is often also accompanied by drug-resistant epilepsy and autism spectrum behavioural disorders. Recently, publications have appeared which attest to the effectiveness of mTOR inhibitors, when the administration thereof is brought forward to under the age of 2, in improving the clinical expression of the latter two aspects as well. But no data have been published to suggest a benefit in cases of cognitive disability.

It should thus also be assessed whether other epilepsies as well, neuropsychiatric disorders and cognitive deficits, which are today either incurable or not treatable in a satisfactory manner, may derive a benefit from therapy with mTOR inhibitors at an appropriate dosage.

In 2011 the effect of the oral administration of mTOR inhibitors on a model of mice affected by Alzheimer's disease was published for the first time; an improvement was observed both in the neurofibrillary tangles composed of Tau protein and extra- and intraneuronal accumulations of beta-amyloid protein in the brain (classic anatomopathological characteristics of Alzheimer's disease), with a recovery of learning and memory. The effect seemed due to the induction of autophagy mediated by the inhibition of mTOR. Autophagy is the metabolic process whereby cells degrade substrates produced by them. The increase in this process induces the degradation of pathological protein accumulations in Alzheimer's. The activity of mTOR inhibits it, whereas the inhibition thereof stimulates it. It is not yet completely understood whether the symptomatologic benefit observed is entirely attributable to this phenomenon.

Despite the benefits clinically observed after the systemic administration of mTOR inhibitors in neuro-oncological diseases (e.g. tuberous sclerosis), and in mouse models of neuroinflammatory diseases (e.g. models of multiple sclerosis), neurodegenerative diseases (e.g. models of Alzheimer's disease) and models of many other neurological diseases, there is a difficulty in reaching effective drug concentrations in the central nervous system. Furthermore, the systemic administration of mTOR inhibitors poses serious problems of toxicity tied to the high biodistribution thereof and thus a considerable immunosuppressant effect, in the case of use in therapies such as the ones mentioned above, represents a major undesirable effect.

*Cerebrospinal fluid* (CSF), the fluid produced by the choroid plexus of the brain ventricles, which fills them and from which it flows out to surround the brain, can be considered an ultrafiltrate of plasma: under physiological conditions, it contains ions, a few proteins and very few cells. If injected here, in the brain ventricles, the drug will be homogeneously distributed in the brain parenchyma.

Its volume varies between 135 and 150 ml, but it is continuously produced at a rate of 500 ml/day. It is absorbed by Pacchioni granulations, protruding formations in the venous sinuses of the dura mater and reintroduced into the bloodstream through venous blood. Therefore, it is entirely replaced about 3.5 times a day.

Consequently, if it is desired to maintain the concentration of a drug in the central nervous system (which has a volume of approx. 1400 ml), it will be necessary to administer it continuously into the cerebrospinal fluid. The continuous turnover with blood circulation produces a systemic absorption of the drug, whose blood concentration will depend on its half-life and distribution volume.

On the basis of these considerations, a therapeutic strategy has been proposed which provides for intrathecal, preferably intraventricular, administration of one or more mTOR inhibitors using intracerebroventricular administration devices. This new therapy has been experimented with on normal and 3xTg-AD mouse models and described in patent EP2846770B1. Intrathecal administration of mTOR inhibitors is also disclosed in Cassano et al., Experimental Neurology (Vol. 311), January 2019, pages 88-105.

However, although they possess a long half-life in bodily fluids, mTOR inhibitors, at body temperature, are unstable molecules that rapidly degrade inside infusion devices. This instability precludes ensuring the sustained effect that should be observed with a gradual administration, above all over a number of weeks. This therefore implies the need for repeated daily administration of mTOR inhibitors, also intrathecally.

It is thus necessary to develop a method capable of drastically increasing the stability of these drugs at body temperature inside infusion devices.

### SUMMARY OF THE INVENTION

A first aspect of the present invention relates to a single-layer lipid vesicle containing at least one mTOR inhibitor for use in the treatment or prevention of a disease of the central nervous system, wherein the vesicle is administered via the intrathecal route, wherein said vesicle comprises one or more phospholipids to which one or more hydrophilic polymers are bound, wherein said phospholipids are selected from: distearoylphosphoethanolamine (DSPE), dipalmitoylphosphatidylcholine (DPPC), distearoylphosphatidylcholine (DSPC), diarachidoylphosphatidylcholine (DAPC), dibenoylphosphatidylcholine (DBPC), ditricosanoylphosphatidylcholine (DTPC) dilignoceroylphosphatidylcholine (DLPC) and combinations thereof, wherein said hydrophilic polymers are selected from: polyethylene glycol (PEG), polyoxazolines, polyvinyl alcohol (PVA), polyglycerol, polyvinylpyrrolidone (PVP), poly-N-hydroxypropyl methacrylamide (polyHPMA), polyaminoacids and combinations thereof, and wherein said at least one mTOR inhibitor is selected from: everolimus, rapamycin, ridaforolimus, temsirolimus and combinations thereof.

A second aspect of the present invention relates to a pharmaceutical composition comprising the above-described lipid vesicles for use as defined above.

The composition is preferably formulated in liquid form, preferably as a suspension.

Throughout the specification references to methods of treatment or prevention of diseases are to be interpreted as compositions for use in said method of treatment or prevention.

Disclosed but not claimed is a method for the treatment or prevention of a neurodegenerative disease or tumoral disease with neurological involvement or a neuroinflammatory disease or a disorder characterised by chronic pain of central origin, which comprises the step of locally administering, into a patient's brain, an effective dose of the lipid vesicles containing at least one mTOR inhibitor or of the composition comprising said vesicles.

### BRIEF DESCRIPTION OF THE FIGURES

The present invention is described below in detail and illustrated by way of example with reference to the appended figures, in which:
**Figure 1** shows the result of an HPLC evaluation of the degradation of a solution of micellar everolimus and of a solution of everolimus in DMSO stored at 37°C, 25°C and 4°C;
**Figure 2** shows an evaluation of cell proliferation by MTT assay in HeLa cells (Figure 2A) and SH-SY5Y cells (Figure 2B) treated for 24h and 48h with empty micelles at increasing concentrations. The data represent the mean ± S.D. of the experiment conducted in quintuplicate.
**Figure 3** shows an evaluation of the residual activity obtained from profiles of the MTT proliferation assay in HeLa (A) and SH-SY5Y (B) cells treated with a solution of everolimus in DMSO and micelles loaded with the drug. The residual activity is expressed as a percentage of inhibition of proliferation compared to the reference standard.
**Figure 4** shows an evaluation of the toxicity of empty DSPE-PEG₂₀₀₀ micelles on HeLa and SH-S5Y5Y cells at 24 hours (A) and 48 hours (B) after the treatment, and the toxicity of empty DSPE-PEG₂₀₀₀ micelles on HeLa cells in PBS and saline solution at 24 hours (C) and 48 hours (D) after the treatment.
**Figure 5** shows the stability of micelles loaded with everolimus in saline solution, PBS and aCSF (artificial cerebrospinal fluid) at 37°C.
**Figure 6** shows the change over time in the polydispersity (PI) of micelles loaded with everolimus in saline solution and PBS.
**Figure 7** shows the change over time in the hydrodynamic dimensions (MHD) of micelles loaded with everolimus in a PBS solution (A) and in a saline solution (B).

### DEFINITIONS

In the context of the present invention, "minimum effective concentration" means the minimum concentration at which the desired biological effect is obtained.

In the context of the present invention, "compliance" means the patient's adherence, after careful physician counselling, to a therapy, generally a pharmacological one.

### DETAILED DESCRIPTION OF THE INVENTION

A first aspect of the present invention relates to a single-layer lipid vesicle containing at least one mTOR inhibitor for use in the treatment or prevention of a disease of the central nervous system, wherein the vesicle is administered via the intrathecal route, wherein said vesicle comprises one or more phospholipids to which one or more hydrophilic polymers are bound, wherein said phospholipids are selected from: distearoylphosphoethanolamine (DSPE), dipalmitoylphosphatidylcholine (DPPC), distearoylphosphatidylcholine (DSPC), diarachidoylphosphatidylcholine (DAPC), dibenoylphosphatidylcholine (DBPC), ditricosanoylphosphatidylcholine (DTPC), dilignoceroylphosphatidylcholine (DLPC) and combinations thereof, wherein said hydrophilic polymers are selected from: polyethylene glycol (PEG), polyoxazolines, polyvinyl alcohol (PVA), polyglycerol, polyvinylpyrrolidone (PVP), poly-N-hydroxypropyl methacrylamide (polyHPMA), polyaminoacids and combinations thereof, and wherein said at least one mTOR inhibitor is selected from: everolimus, rapamycin, ridaforolimus, temsirolimus and combinations thereof. The disease is preferably a neurodegenerative disease or a tumoral disease with neurological involvement or a neuroinflammatory disease or drug-resistant epilepsy, preferably *West syndrome*, or a neurobehavioural autism spectrum disorder, or a disorder characterised by chronic pain of central origin.

Preferably, the neurodegenerative disease is selected from Alzheimer's disease, Parkinson's disease, Huntington's chorea, frontotemporal dementia, vascular dementia, preferably of the genetically determined CADASIL type (Cerebral Autosomal Dominant Arteriopathy with Subcortical Infarcts and Leukoencephalopathy), prion encephalopathy and amyotrophic lateral sclerosis. In a preferred embodiment, the neurodegenerative disease is Alzheimer's disease.

The tumoral disease with neurological involvement is preferably tuberous sclerosis (TSC).

The neuroinflammatory disease is preferably selected from: multiple sclerosis (MS), acute disseminated encephalomyelitis (ADEM), Devic's neuromyelitis optica, Rasmussen's syndrome, primary CNS vasculitis and primary progressive aphasia.

The at least one mTOR inhibitor is selected from: everolimus, rapamycin (sirolimus), ridaforolimus temsirolimus and combinations thereof.

In a preferred embodiment of the invention, the at least one mTOR inhibitor is selected from: everolimus, rapamycin and combinations thereof.

The lipid vesicles are in a single layer.

The single-layer lipid vesicles most widely available and used in the biomedical field, above all as carriers for drugs or other molecules of biological or therapeutic importance, are "micelles". In one embodiment of the invention, therefore, the single-layer lipid vesicles containing at least one mTOR inhibitor can preferably be micelles. As is well known in the sector, micelles are formed from at least one layer of one or more phospholipids.

The lipid layer of the vesicles described here, preferably of the micelles, can consist essentially of one or more saturated phospholipids; in another embodiment, the layer can consist essentially of one or more unsaturated phospholipids. More typically, however, the layer can consist essentially of a mixture of saturated phospholipids and unsaturated phospholipids.

The one or more phospholipids forming the layer of the lipid vesicles are selected from the group consisting of: distearoylphosphoethanolamine (DSPE), dipalmitoylphosphatidylcholine (DPPC), distearoylphosphatidylcholine (DSPC), diarachidoylphosphatidylcholine (DAPC), dibenoylphosphatidylcholine (DBPC), ditricosanoylphosphatidylcholine (DTPC), dilignoceroylphosphatidylcholine (DLPC) and combinations thereof.

In a preferred embodiment, the layer of single-layer lipid vesicles, preferably micelles, comprises distearoylphosphatidylethanolamine (DSPE).

The lipids forming the lipid layer are modified by binding one or more hydrophilic polymers to them. Typically, one or more hydrophilic polymers can be bound to the polar heads of the phospholipids present in the layer of vesicles, preferably micelles. In other words, the vesicles can carry one or more hydrophilic polymers on their outer surface, typically bound to the polar heads of the phospholipids.

The presence of hydrophilic polymers on the outer surface of the vesicles imparts a longer half-life following parenteral administration. In particular, one or more hydrophilic polymers prevent (or at least render more difficult) the uptake of the vesicles by the reticuloendothelial system, since they form an external barrier that prevents (or at least renders more difficult) the adsorption of plasma proteins by the vesicles themselves. The presence of the one or more hydrophilic polymers on the outer surface thus increases the stability of the vesicles, ensuring that they remain longer in the physiological fluids after administration.

The one or more hydrophilic polymers selected from the group consisting of: polyethylene glycol (PEG), polyoxazolines, polyvinyl alcohol (PVA), polyglycerol, polyvinylpyrrolidone (PVP), poly-N-hydroxypropyl methacrylamide (polyHPMA), polyaminoacids and combinations thereof.

In a preferred embodiment of the invention, the hydrophilic polymer bound to the phospholipids of the lipid vesicles is polyethylene glycol (PEG).

In one embodiment, the PEG is selected from: PEG₁₀₀₀, PEG₂₀₀₀, PEG ₃₄₀₀ and PEG₅₀₀₀, preferably PEG₂₀₀₀.

In a preferred embodiment of the invention, the lipid vesicles, preferably the micelles, comprise the phospholipid DSPE bound to the hydrophilic polymer PEG, preferably to the hydrophilic polymer PEG₂₀₀₀.

In one embodiment, the molar ratio between the mTOR inhibitor and vesicle is comprised between 1:5 and 1:10, preferably 1:7.

The vesicles of the present invention are preferably prepared by mixing the at least one mTOR inhibitor with at least one phospholipid forming the layer of lipid vesicles. In one embodiment, at least one mTOR inhibitor and at least one phospholipid are weighed and dissolved in a solvent. The solvent is later removed, preferably by evaporation and/or drying.

The vesicles are then resuspended in an aqueous medium in order to obtain a clear micellar dispersion.

In one embodiment, after their preparation the vesicles are prepared under aseptic conditions using any suitable method, for example after sterilisation of the receptacles in an autoclave and of the solutions by filtration.

The vesicles are administered via the intrathecal route, more preferably via the intraventricular route. Intrathecal administration can be performed in a pulsed manner, preferably at regular intervals, or in a continuous manner.

Intrathecal administration is preferably performed by means of a mechanical or electromechanical device, more preferably by means of an electromechanical infusion pump. The mechanical or electromechanical device can be, for example, an implanted device, i.e. surgically implanted under the scalp and provided with a subcutaneous reservoir, leading off from which there is a catheter extending from the cranial vault until reaching the intraventricular space (similar to an "Ommaya Reservoir"). Alternatively, a pump can be inserted beneath the skin (so-called "ports") in the periclavicular region, or implanted in the abdomen (infusion pumps). Connected to the pump there is a catheter, which is inserted into the intrathecal space, or reaches the ventricular space, first via the subcutaneous route, then the transcranial route.

The use of micelles for the diffusion of drugs into the central nervous system (CNS) is usually not considered a suitable means, as the ability of micelles to pass through the ependymal barrier (very similar to the blood-brain barrier) is entrusted to the small portion thereof involved in transcytotis.

Surprisingly, the Applicant has demonstrated that incorporating the mTOR inhibitor in lipid vesicles, makes it possible to reach a minimum effective concentration of mTOR inhibitors in CSF. In fact, without wishing to be bound to any theory, at body temperature, in a low presence of solutes (under normal conditions and generally in the pathological conditions we intend to treat, CFS is a plasma ultrafiltrate with very low protein and cell concentrations) and below their critical micellar concentration, lipid vesicles, in particular micelles, invariably disintegrate, releasing the drug in the medium. In CSF, the micelles are at a critically low concentration and will disintegrate. Being lipophilic molecules, mTOR inhibitors in CSF can thus freely disseminate in the brain parenchyma. Once in CSF, they are homogeneously diffused in the subarachnoid space, penetrate into the parenchyma, and reach and enter effectively into the target cells.

Furthermore, intrathecal administration makes it possible to avoid the side effects due to systemic administration of mTOR inhibitors. In fact, besides increasing the concentration of mTOR inhibitors in CSF, intrathecal administration prevents the mTOR inhibitors from reaching high concentrations in other regions of the body, reducing the risk of immunosuppressant effects.

Intrathecal administration of mTOR inhibitors, together with the incorporation thereof into lipid vesicles, is capable of increasing both the concentration of mTOR inhibitors in CSF and the stability thereof. In fact, as demonstrated in the experimental section of the present application, micelles increase the stability of mTOR inhibitors at different temperatures; in particular, at 37°C the everolimus loaded in the micelles demonstrated a much higher stability than everolimus in solution in DMSO.

Intrathecal administration of mTOR inhibitors encapsulated in micelles could thus demonstrate to be a very effective therapy for very severe neurological pathologies, such as, for example, neurodegenerative diseases, tumoral diseases with neurological involvement and neuroinflammatory diseases, while avoiding systemic side effects and enabling a greater stability and concentration of the drug in CSF. In fact, the greater stability of mTOR inhibitors encapsulated in lipid vesicles, preferably in micelles, would enable more extended administrations of the mTOR inhibitors and fewer systemic side effects, thus increasing patient *compliance* with the therapy.

For the purposes of the above-described uses, the vesicles can preferably be administered at least once or twice a month, the infusion phase lasting 2-3 weeks, thus allowing for 1-2 week break between 2 administrations.

In a preferred embodiment, the vesicles are administered in a single administration or in a number of administrations on a monthly basis or at different time internals according to need.

A second aspect of the present invention relates to a composition comprising the vesicles as described above in detail for use as defined above. $

The pharmaceutical composition of the invention can be formulated with suitable excipients known to the person skilled in the art.

According to one embodiment of the invention, the composition is formulated in liquid form, preferably as a suspension.

Alternatively, the composition is prepared extemporaneously by mixing the lyophilised and/or dry composition with a saline solution, water or, preferably, aCSF.

The composition is administered via the intrathecal route, more preferably via the intraventricular route.

Intrathecal administration can be performed in a pulsed manner, preferably at regular intervals, or in a continuous manner.

Intrathecal administration is preferably performed by means of a mechanical or electromechanical device, more preferably by means of an electromechanical infusion pump. The mechanical or electromechanical device can be, for example, an implanted device, i.e. surgically implanted under the scalp and provided with a subcutaneous reservoir, leading off from which there is a catheter extending from the cranial vault until reaching the intraventricular space (similar to an "Ommaya Reservoir").

Alternatively, a pump can be inserted beneath the skin (so-called "ports") in the periclavicular region, or implanted in the abdomen (infusion pumps). Connected to the pump there is a catheter, which is inserted into the intrathecal space, or reaches the ventricular space, first via the subcutaneous route, then the transcranial route.

Disclosed but not claimed is a method for the treatment and/or the prevention of a pathology of the central nervous system, preferably a disease of the central nervous system, preferably a neurodegenerative disease or a tumoral disease with neurological involvement or a neuroinflammatory disease or a drug-resistant epilepsy or a neurobehavioural autism spectrum disorder of unknown origin or a disorder characterised by chronic pain of central origin, which comprises a step of administering to the patient an effective dose of the vesicles of the present invention as described above in detail or of the composition comprising the vesicles.

Preferably, the neurodegenerative disease is selected from Alzheimer's disease, Parkinson's disease, Huntington's chorea, frontotemporal dementia, vascular dementia, preferably of the genetically determined CADASIL type (Cerebral Autosomal Dominant Arteriopathy with Subcortical Infarcts and Leukoencephalopathy), prion encephalopathy and amyotrophic lateral sclerosis. Preferably, the neurodegenerative disease is Alzheimer's disease.

The tumoral disease with neurological involvement is preferably tuberous sclerosis (TSC).

The neuroinflammatory disease is preferably selected from: multiple sclerosis (MS), acute disseminated encephalomyelitis (ADEM), Devic's neuromyelitis optica, Rasmussen's syndrome, primary CNS vasculitis and primary progressive aphasia.

The vesicles are administered via the intrathecal route, more preferably via the intraventricular route. Intrathecal administration can be performed in a pulsed manner, preferably at regular intervals, or in a continuous manner.

Intrathecal administration is preferably performed by means of a mechanical or electromechanical device, more preferably by means of an electromechanical infusion pump.

The mechanical or electromechanical device can be, for example, an implanted device, i.e. surgically implanted under the scalp and provided with a subcutaneous reservoir, leading off from which there is a catheter extending from the cranial vault until reaching the intraventricular space (of the "Ommaya Reservoir" type). Alternatively, a pump can be inserted beneath the skin (so-called "ports") in the periclavicular region, or implanted in the abdomen (infusion pumps). Connected to the pump there is a catheter, which is inserted into the intrathecal space, or reaches the ventricular space, first via the subcutaneous route, then the transcranial route.

In a preferred embodiment, the vesicles are administered in a single administration or in a number of administrations lasting 15 days, with a 15 day break in between, until remission of the symptoms and normalisation of the disease marker.

### Example

### PREPARATION OF THE EVEROLIMUS SOLUTION IN AN ORGANIC SOLVENT (REFERENCE COMPOSITION)

Everolimus (Selleck Chemicals, Aurogene, Rome, Italy) was dissolved in DMSO (VWR, Milan, Italy) at a concentration of 1 mg/ml. The everolimus solution was subsequently diluted in 10% v/v DMSO/saline solution until obtaining a final concentration of 10 µg/ml. The resulting solution was then incubated at 4, 25 and 37°C.

### PREPARATION MICELLES LOADED WITH EVEROLIMUS

Carefully weighed amounts of everolimus and distearoylphosphoethanolamine-polyethylene glycol 2000 (DSPE-PEG2000, Lipoid, Switzerland) were dissolved in 3 ml of chloroform (Sigma-Aldrich, Milan, Italy) in a 50 ml flask. The solvent was removed by light evaporation at room temperature under a flow of nitrogen and vacuum dried for 1 hour to eliminate residual traces. The thin film obtained was reconstituted in an exact volume (3 mL) of aqueous medium. After complete reconstitution a clear micellar dispersion was obtained. The dispersion was then incubated at 4, 25 and 37°C.

### QUANTITATIVE ANALYSIS OF EVEROLIMUS

The drug was quantified using the HPLC method. Briefly, the instrument used is a Portlab STAYER HPLC system equipped with a UV detector, a parallel pump and Triathlon autosampler (Portlab, Rome, Italy). The conditions used are the following: isocratic mode with acetonitrile:water (60:40, v/v) eluted at 0.8 ml/min and a Zorbax C8 column, 4.6 mm ID x 250 mm, 5 µm (Agilent, Milan, Italy) equilibrated at 55°C. UV detection was performed at 278 nm. A calibration curve was constructed in the concentration interval of 0.62-10 µg/mL (r² = 0.9992).

The samples of solution were injected directly (injection volume 15 µL), whereas the micellar suspension was diluted 200 times in DMSO before being subjected to analysis. In order to reduce the *carryover* effects and an excessive accumulation of lipids in the column, samples of solution and micelles were injected alternately. The column was periodically washed with chloroform and methanol to avoid the accumulation of lipids and impairment of performance. All measurements were performed in triplicate and the results were expressed as the mean ±D.S.

### ANALYSIS OF PH AND EFFECT OF THE MEDIUM

The role of pH and the effect of the medium on the stability of the micelles loaded with everolimus were analysed by incubating the samples in different media at 37°C. Saline solution, 10 mM phosphate-buffered saline, pH 7.4 (PBS, Sigma-Aldrich, Milan, Italy) and artificial cerebrospinal fluid (aCSF) were used as the incubation solution.

### Dimensional analysis

Measurements of size distribution were performed using a Nicomp 380 ZLS autocorrelator (PSS Inc., Santa Barbara, CA, USA) equipped with a Coherent Innova 70-3 He-Ne laser (Laser Innovation, Moonpark, CA, USA), an avalanche photodiode detector (APD) and a PALS phase analyser. The polydispersity index (PI) and the mean hydrodynamic diameter (MHD) of the micelles loaded with everolimus in saline solution and in PBS were measured in triplicate.

### CELL CULTURES

Both HeLa cells (cervical cancer cell line) and SH-SY5Y cells (neuroblastoma cell line) were obtained from the American Type Culture Collection (Manassas, VA, USA) and cultured in Dulbecco's Modified Eagle's medium (DMEM with L-glutamine) supplemented with 10% (v/v) heat-inactivated foetal bovine serum (FBS), 100 U of penicillin, 100 U of streptomycin in a 5% CO₂ humidified incubator at 37°C. Cell viability was verified with the standard Trypan Blue method.

### ANALYSIS WITH MTT COLORIMETRIC ASSAY

The activity of everolimus in organic solution and in the micelles was determined by evaluating cell proliferation after the treatment. Briefly, the HeLa and SH-SY5Y cells were seeded in a 96-well plate at a concentration of 3000 cells/well and 6000 cells/well, respectively, and were maintained in the culture medium (100 µl) for 24 hours. After incubation at 37, 25 and 4°C for 7, 14, 35, 50 and 77 days, the samples were suspended in the cell culture medium at a final concentration of 5 nM. Untreated cells, cells treated with a 10% v/v DMSO/saline solution (Veh) and empty micelles in saline solution, both diluted in a manner equivalent to the drug, were analysed as controls. A freshly prepared everolimus solution was used as the reference standard. Cell proliferation was evaluated using the 3-[4,5-dimethylthiazol-2-yl] -2,5-diphenyltetrazolium (MTT) assay (Sigma-Aldrich) according to the manufacturer's manual. The cells were mixed with 10 µl of MTT (0.5 mg/ml) and kept in a humidified incubator at 37°C for 4 hours. Subsequently, 100 µl of a solubilisation solution (10% SDS with 0.01 N HCl) were added to each well. Formazan crystal solubilisation was carried out overnight in an incubator a 37°C. The next day, cell proliferation was evaluated by means of spectrophotometric readings taken with a Beckman Coulter DTX 880 multimode detector (Beckman Coulter Inc., Brea, California, USA) using a line at 589 nm and reference at 650 nm.

### CELL TOXICITY

The toxicity of the micelles was evaluated in HeLa cells by means of the MTT assay. The HeLa cells were seeded in a 96-well plate at a concentration of 3000 cells/well and maintained in 100 µL of medium. The test was carried out after 24 and 48 hours of treatments. The empty micelles, suspended in PBS or in saline solution, were used at a final concentration of 0, 2, 1, 2, 4, 8, 16 and 32X, in reference to the concentration of the loaded micelles, 5 nM, considered as 1X.

### EVALUATION OF STABILITY BY HPLC ANALYSIS

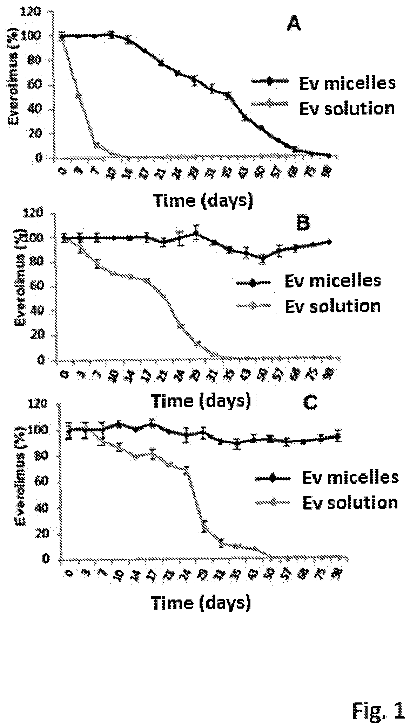

At 37°C, the micelles loaded with everolimus showed much higher stability than the solution, as shown in Figure 1A. In fact, whereas the solution had fallen to zero by day 14, the micelles were detectable until day 98. As predicted, all of the samples were considerably more stable at 25 and 4°C than at 37°C, but with a clear superiority of the micelles (Figures 1B and 1C). Under these conditions, the micelles loaded with everolimus remained practically unchanged throughout the entire study period, whereas the solutions had decayed by days 35 and 50, at 25 and 4°C respectively.

### EVALUATION OF THE ACTIVITY OF EVEROLIMUS ON HeLa AND SH-SY5Y CELLS

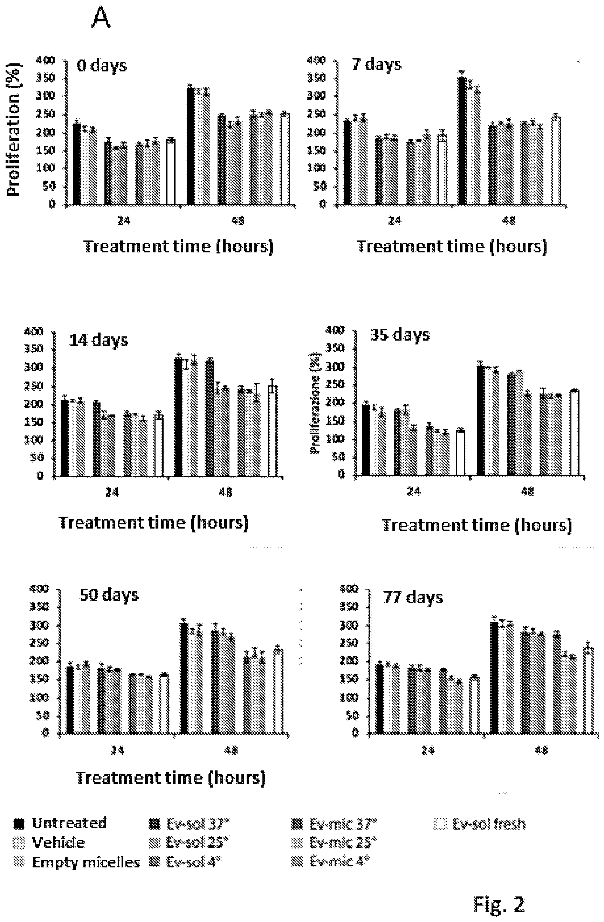

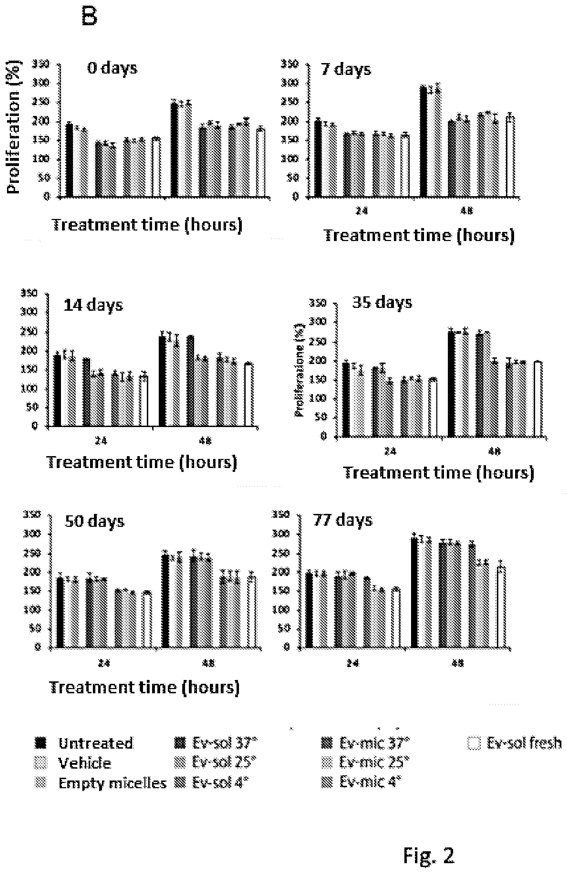

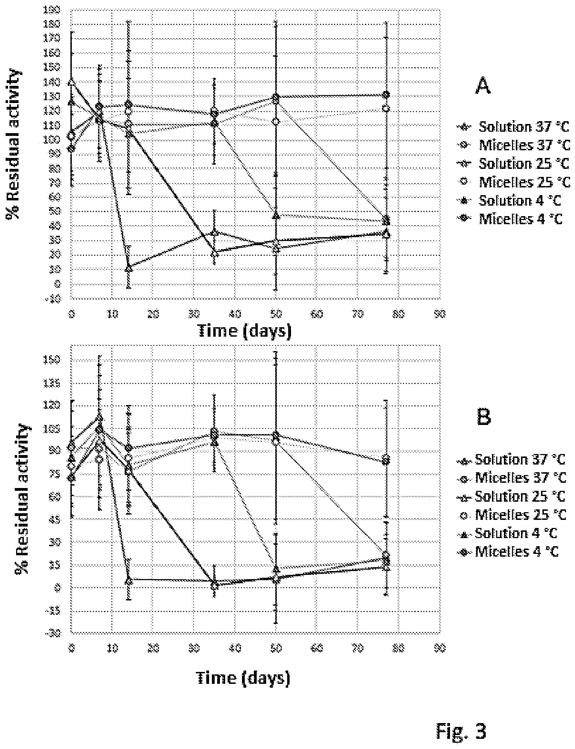

In order to correlate the stability of everolimus with the preservation of activity, samples incubated under the same conditions as in the stability study were dosed in HeLa and SH-SY5Y cells at established points in time. For this test the cell lines were seeded and maintained for 24 hours at 37°C prior to treatment. The solution and the micelles loaded with everolimus were tested at a final concentration of 5 nM. As shown in Figures 2A and 2B, at 37°C the effect of the micelles (37, 25 and 4°C) remained unchanged after 77 days of treatment. The activity of the solution, by contrast, began to wane at 35 days, confirming a prolonged effect of inhibition of the micelles on cell proliferation. The results were comparable in both cell lines. In order to better quantify the differences among the various samples in terms of the residual activity of everolimus, the residual anti-proliferative activity was calculated and is shown in Figures 3A and 3B.

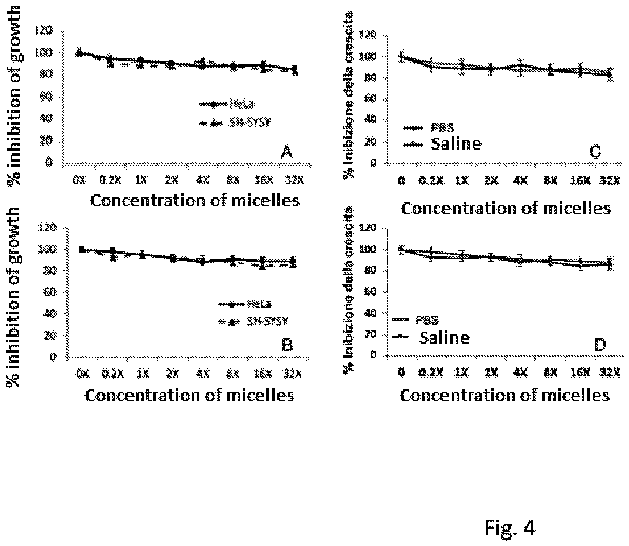

The solutions clearly showed a drastic decrease in activity at 14, 35 and 50 days, at 37, 25 and 4°C, respectively, data that correlate perfectly with the instability previously observed. All of the micelles, by contrast, were highly stable, remaining completely active at 25 and 4°C, whereas the ones incubated at 37°C decreased to about 20% at 77 days. These results demonstrate a perfect correspondence between the stability measured and the activity obtained without any difference between the two cell lines. In order to determine the biocompatibility in vitro of the DSPE-PEG₂₀₀₀ micelles, the toxicity of the empty micelles was evaluated in the HeLa and SH-SY5Y cell lines by MTT analysis. The empty micelles, suspended in PBS or in saline solution, were used at a final concentration of 0, 0,2, 1, 2, 4, 8, 16 and 32X, where 1X = equivalent to 5 nM of everolimus. As shown in Figures 4A, 4B, cell proliferation was not influenced by the treatment with micelles even at the maximum concentration used, 32 times the initial one. Both cell lines showed the same tolerance to the treatment at both times. The replacement of PBS with saline solution did not have any significant effects (Fig. 4C, 4D).

Overall, the DSPE-PEG₂₀₀₀ micelles seem to be highly tolerated and safe, at least in vitro, supporting their clinical application.

The evaluation of the stability of DPSE-PEG₂₀₀₀ micelles loaded with everolimus in different media at 37°C revealed some important differences that are useful for establishing clinical conditions optimal for use of the formulation.

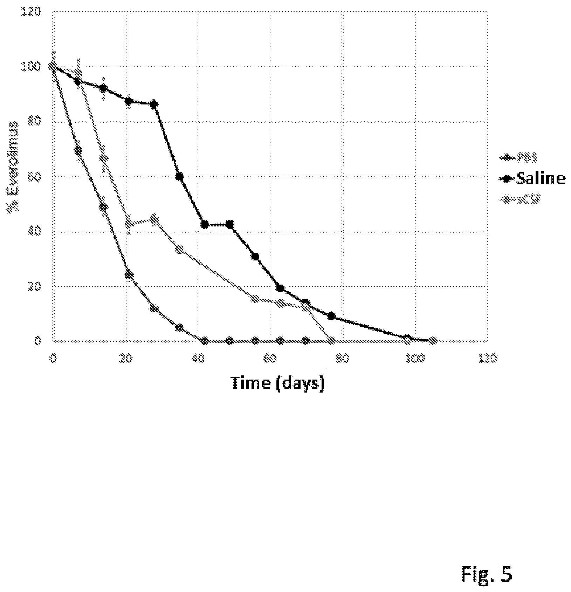

In Figure 5 the profiles obtained clearly show a reduced stability of the micelles in PBS compared to saline solution or aCSF, which was used to approximately imitate the physiological environment.

The percentage of Everolimus loaded in micelles in PBS decreased until arriving at zero at day 42, whereas the stability in aCSF was nearly double up to day 80. The behaviour in saline solution was nearly completely identical to that observed during the first stability study, which confirmed the already observed stabilisation capacity of the micelles. The high stability in aCSF may suggest a high compatibility with bodily fluids during prolonged administration of the formulation. Naturally, a further destabilisation is to be expected in bodily fluids due to the enzymatic component, which was not taken into consideration in this simulation, but which we predict to be negligible in CSF.

Furthermore, the variations in the size distribution of the micelles over time in PBS and saline solution were studied.

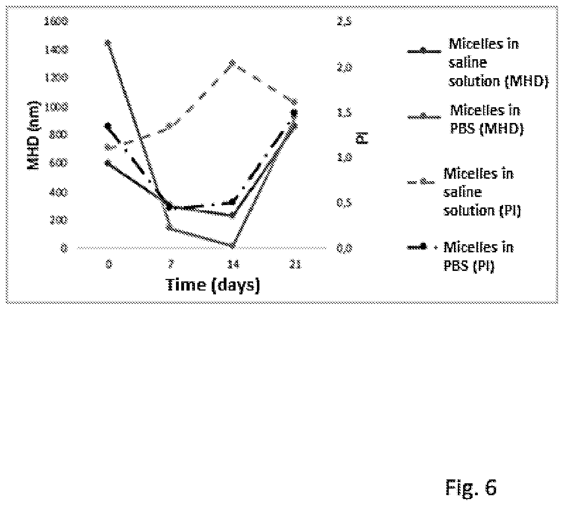

Figure 6 shows the MHD profiles of the size distributions of the micelles up to 21 days of incubation. Initially, MHD was higher and decreased progressively up to day 14, to about 200 nm and 30 nm in saline solution and PBS, respectively.

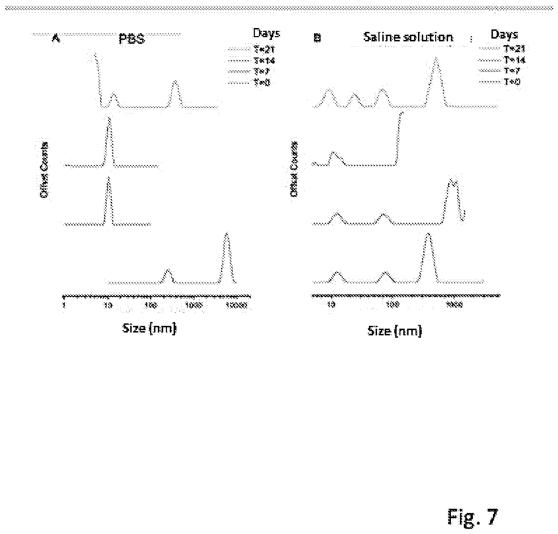

This behaviour is explained by an initial period of organisation of the micelles, which led to a size adjustment in both media. A similar trend was observed for the value of the polydispersity index (PI) in PBS, whereas PI remained more constant in saline solution. In general, the micellar dispersion is finer and more homogeneous in PBS with a PI around 0.4 compared to a PI of about 1.3 in saline solution (Figure 7). However, a much more rapid destabilisation was observed in PBS, as also demonstrated by an abrupt increase in MHD and PI at 21 days. This effect was attributed to phosphate anions, which might be capable of disrupting the micellar structure, perhaps due to interaction of charge, causing a loss in the ability to protect the drug from hydrolysis.

These results, together with the size distribution profiles, suggest that the high stabilisation observed is probably due to an aggregate state of the micellar dispersion in the saline solution. In PBS, by contrast, this aggregate state is disrupted by the presence of phosphate with a consequent reduction in the protective ability of the micelles over time.

### Conclusions

The micellar solution of everolimus is more stable than the solution of everolimus in organic solvent, as it is more slowly degraded. In fact, the results obtained from the HPLC assays and MTT assays show that the micellar solution of everolimus is capable of maintaining the molecule of everolimus stable for a number of days, thus improving the effectiveness thereof (Figure 1). As positive results were obtained in vitro, they can be repeated with rapamycin and further analyses can be conducted, centred on the use of the micellar solution of everolimus/rapamycin administered in vivo, in normal and AD mouse models and then in patients. This will serve to provide a more complete understanding of the mechanism of action of the micellar solution of mTOR inhibitors and the effect thereof on AD.

## Claims

1. A single-layer lipid vesicle containing at least one mTOR inhibitor for use in the treatment or prevention of a disease of the central nervous system, wherein the vesicle is administered via the intrathecal route, wherein said vesicle comprises one or more phospholipids to which one or more hydrophilic polymers are bound,
wherein said phospholipids are selected from: distearoylphosphoethanolamine (DSPE), dipalmitoylphosphatidylcholine (DPPC), distearoylphosphatidylcholine (DSPC), diarachidoylphosphatidylcholine (DAPC), dibenoylphosphatidylcholine (DBPC), ditricosanoylphosphatidylcholine (DTPC), dilignoceroylphosphatidylcholine (DLPC) and combinations thereof,
wherein said hydrophilic polymers are selected from: polyethylene glycol (PEG), polyoxazolines, polyvinyl alcohol (PVA), polyglycerol, polyvinylpyrrolidone (PVP), poly-N-hydroxypropyl methacrylamide (polyHPMA), polyaminoacids and combinations thereof,
and wherein said at least one mTOR inhibitor is selected from: everolimus, rapamycin, ridaforolimus, temsirolimus and combinations thereof.

2. The vesicle for use according to claim 1, wherein the vesicle is administered via the intraventricular route.

3. The lipid vesicle for use according to claim 1 or 2, wherein said single-layer vesicle is a micelle.

4. The vesicle for use according to any one of claims 1-3, wherein the one or more hydrophilic polymers bound to phospholipids is polyethylene glycol (PEG).

5. The vesicle for use according to any one of claims1-4, wherein the disease of the central nervous system is a neurodegenerative disease or a tumoral disease with neurological involvement or a neuroinflammatory disease or drug-resistant epilepsy or a neurobehavioural autism spectrum disorder of unknown origin.

6. The vesicle for use according to claim 5, wherein the neurodegenerative disease is selected from Alzheimer's disease, Parkinson's disease, Huntington's chorea, frontotemporal dementia, vascular dementia, preferably of the genetically determined CADASIL type, prion encephalopathy and amyotrophic lateral sclerosis, the tumoral disease with neurological involvement is tuberous sclerosis (TSC), the neuroinflammatory disease is selected from multiple sclerosis (MS), acute disseminated encephalomyelitis (ADEM), Devic's neuromyelitis optica, Rasmussen's syndrome, opsoclonus-myoclonus syndrome, preferably Kinsbourne syndrome, primary CNS vasculitis and primary progressive aphasia, the drug-resistant epilepsy is West syndrome.

7. The vesicle for use according to claim 6, wherein the neurodegenerative disease is Alzheimer's disease.

8. The vesicle for use according to any one of claims 1-7, wherein the at least one mTOR inhibitor is selected from: everolimus, rapamycin and combinations thereof.

9. The vesicle for use according to any one of claims 1-8, wherein the at least one mTOR inhibitor is everolimus.

10. The vesicle for use according to any one of claims 1-9, wherein the inhibitor: lipid vesicle molar ratio is comprised between 1:5 and 1:10.

11. The vesicle for use according to claim 10, wherein the inhibitor: lipid vesicle molar ratio is 1:7.

12. A pharmaceutical composition comprising the lipid vesicle for use according to any one of claims 1-11.

13. The pharmaceutical composition for use according to claim 12 in liquid form, or in lyophilised and/or dry form reconstitutable in a saline solution.

14. The composition for use according to claim 13, in the form of a suspension or in lyophilised and/or dry form reconstitutable in artificial cerebrospinal fluid (aCSF).

15. The composition for use according to claim 14, in the form of a dry powder reconstitutable in aCSF.

## Patentansprüche

1. Einschichtiges Lipidvesikel, das mindestens einen mTOR-Hemmer enthält, zur Verwendung bei der Behandlung oder Vorbeugung einer Erkrankung des zentralen Nervensystems, wobei das Vesikel über den intrathekalen Weg verabreicht wird, wobei das Vesikel ein oder mehrere Phospholipide umfasst, an die ein oder mehrere hydrophile Polymere gebunden sind,
wobei die Phospholipide ausgewählt sind aus: Distearoylphosphoethanolamin (DSPE), Dipalmitoylphosphatidylcholin (DPPC), Distearoylphosphatidylcholin (DSPC), Diarachidoylphosphatidylcholin (DAPC), Dibenoylphosphatidylcholin (DBPC), Ditricosanoylphosphatidylcholin (DTPC), Dilignoceroylphosphatidylcholin (DLPC) und Kombinationen davon,
wobei die hydrophilen Polymere ausgewählt sind aus: Polyethylenglykol (PEG), Polyoxazoline, Polyvinylalkohol (PVA), Polyglycerol, Polyvinylpyrrolidon (PVP), Poly-N-hydroxypropylmethacrylamid (PolyHPMA), Polyaminosäuren und Kombinationen davon,
und wobei der mindestens eine mTOR-Hemmer ausgewählt ist aus: Everolimus, Rapamycin, Ridaforolimus, Temsirolimus und Kombinationen davon.

2. Vesikel zur Verwendung nach Anspruch 1, wobei das Vesikel über den intraventrikulären Weg verabreicht wird.

3. Lipidvesikel zur Verwendung nach Anspruch 1 oder 2, wobei das einschichtige Vesikel eine Mizelle ist.

4. Vesikel zur Verwendung nach einem der Ansprüche 1-3, wobei das eine oder die mehreren hydrophilen Polymere, die an Phospholipide gebunden sind, Polyethylenglykol (PEG) ist.

5. Vesikel zur Verwendung nach einem der Ansprüche 1-4, wobei die Erkrankung des zentralen Nervensystems eine neurodegenerative Erkrankung oder eine Tumorerkrankung mit neurologischer Beteiligung oder eine neuroinflammatorische Erkrankung oder arzneimittelresistente Epilepsie oder eine neurobehaviorale Autismus-Spektrum-Störung unbekannten Ursprungs ist.

6. Vesikel zur Verwendung nach Anspruch 5, wobei die neurodegenerative Erkrankung ausgewählt ist aus Alzheimer-Krankheit, Parkinson-Krankheit, Chorea Huntington, frontotemporaler Demenz, vaskulärer Demenz, vorzugsweise vom genetisch bestimmten CADASIL-Typ, Prionenenzephalopathie und amyotropher Lateralsklerose, die Tumorerkrankung mit neurologischer Beteiligung tuberöse Sklerose (TSC) ist, die neuroinflammatorische Erkrankung ausgewählt ist aus multipler Sklerose (MS), akuter disseminierter Enzephalomyelitis (ADEM), Devic-Neuromyelitis-optica, Rasmussen-Syndrom, Opsoclonus-Myoclonus-Syndrom, vorzugsweise Kinsbourne-Syndrom, primärer ZNS-Vaskulitis und primär progredienter Aphasie, die arzneimittelresistente Epilepsie West-Syndrom ist.

7. Vesikel zur Verwendung nach Anspruch 6, wobei die neurodegenerative Erkrankung die Alzheimer-Krankheit ist.

8. Vesikel zur Verwendung nach einem der Ansprüche 1-7, wobei der mindestens eine mTOR-Hemmer ausgewählt ist aus: Everolimus, Rapamycin und Kombinationen davon.

9. Vesikel zur Verwendung nach einem der Ansprüche 1-8, wobei der mindestens eine mTOR-Hemmer Everolimus ist.

10. Vesikel zur Verwendung nach einem der Ansprüche 1-9, wobei das molare Verhältnis von Hemmer zu Lipidvesikel zwischen 1:5 und 1:10 liegt.

11. Vesikel zur Verwendung nach Anspruch 10, wobei das molare Verhältnis von Hemmer zu Lipidvesikel 1:7 beträgt.

12. Pharmazeutische Zusammensetzung, die das Lipidvesikel zur Verwendung nach einem der Ansprüche 1-11 umfasst.

13. Pharmazeutische Zusammensetzung zur Verwendung nach Anspruch 12 in flüssiger Form oder in lyophilisierter und/oder trockener Form, die in einer Salzlösung rekonstituierbar ist.

14. Zusammensetzung zur Verwendung nach Anspruch 13 in Form einer Suspension oder in lyophilisierter und/oder trockener Form, rekonstituierbar in künstlicher Zerebrospinalflüssigkeit (aCSF).

15. Zusammensetzung zur Verwendung nach Anspruch 14 in Form eines Trockenpulvers, das in aCSF rekonstituierbar ist.

## Revendications

1. Vésicule lipidique monocouche, contenant au moins un inhibiteur de mTOR pour le traitement ou la prévention d'une maladie du système nerveux central, dans laquelle la vésicule est administrée par voie intrathécale, dans laquelle ladite vésicule comprend un ou plusieurs phospholipides auxquels sont liés un ou plusieurs polymères hydrophiles,
dans laquelle lesdits phospholipides sont choisis parmi : distéaroyl phosphoéthanolamine (DSPE), dipalmitoyl phosphatidylcholine (DPPC), distéaroyl phosphatidylcholine (DSPC), diarachidoyl phosphatidylcholine (DAPC), dibénoyl phosphatidylcholine (DBPC), ditricosanoyl phosphatidylcholine (DTPC), dilignoceroyl phosphatidylcholine (DLPC) et leurs combinaisons,
dans laquelle lesdits polymères hydrophiles sont choisis parmi : polyéthylène glycol (PEG), polyoxazolines, alcool polyvinylique (PVA), polyglycérol, polyvinylpyrrolidone (PVP), poly-N-hydroxypropylméthacrylamide (polyHPMA), polyaminoacides et leurs combinaisons,
et dans laquelle ledit au moins un inhibiteur de mTOR est choisi parmi : l'évérolimus, rapamycine, ridaforolimus, temsirolimus et leurs combinaisons.

2. Vésicule à utiliser selon la revendication 1, dans laquelle la vésicule est administrée par voie intraventriculaire.

3. Vésicule lipidique à utiliser selon la revendication 1 ou 2, dans laquelle la vésicule monocouche est une micelle.

4. Vésicule à utiliser selon l'une quelconque des revendications 1-3, dans laquelle l'un ou plusieurs des polymères hydrophiles liés aux phospholipides est/sont du polyéthylène glycol (PEG).

5. Vésicule à utiliser selon l'une quelconque des revendications 1-4, dans laquelle la maladie du système nerveux central est une maladie neurodégénérative ou une maladie tumorale avec atteinte neurologique ou une maladie neuroinflammatoire ou une épilepsie pharmacorésistante ou un trouble neurocomportemental du spectre autistique d'origine inconnue.

6. Vésicule à utiliser selon la revendication 5, dans laquelle la maladie neurodégénérative est choisie parmi la maladie d'Alzheimer, la maladie de Parkinson, la chorée de Huntington, la démence frontotemporale, la démence vasculaire, de préférence du type CADASIL déterminé génétiquement, l'encéphalopathie à prions et la sclérose latérale amyotrophique, la maladie tumorale avec atteinte neurologique est la sclérose tubéreuse de Bourneville, la maladie neuroinflammatoire est choisie parmi la sclérose en plaques (SEP), encéphalite aiguë post-infectieuse, la neuromyélite optique, le syndrome de Rasmussen, le syndrome opsoclonus-myoclonus, de préférence le syndrome de Kinsbourne, la vascularite primaire du SNC et l'aphasie progressive primaire, l'épilepsie pharmacorésistante est le syndrome de West.

7. Vésicule à utiliser selon la revendication 6, dans laquelle la maladie neurodégénérative est la maladie d'Alzheimer.

8. Vésicule à utiliser selon l'une quelconque des revendications 1-7, dans laquelle l'au moins un inhibiteur de mTOR est choisi parmi : l'évérolimus, rapamycine et leurs combinaisons.

9. Vésicule à utiliser selon l'une quelconque des revendications 1-8, dans laquelle l'au moins un inhibiteur de mTOR est l'évérolimus.

10. Vésicule à utiliser selon l'une quelconque des revendications 1-9, dans laquelle le rapport molaire inhibiteur/vésicule lipidique est compris entre 1:5 et 1:10.

11. Vésicule à utiliser selon la revendication 10, dans laquelle le rapport molaire inhibiteur/vésicule lipidique est de 1:7.

12. Composition pharmaceutique, comprenant la vésicule lipidique utilisée selon l'une quelconque des revendications 1-11.

13. Composition pharmaceutique à utiliser selon la revendication 12 sous forme liquide, ou sous forme lyophilisée et/ou sèche reconstituable dans une solution saline.

14. Composition pharmaceutique selon la revendication 13, sous forme de suspension ou sous forme lyophilisée et/ou sèche reconstituable dans le liquide céphalorachidien artificiel (LCRa).

15. Composition à utiliser selon la revendication 14, sous forme d'une poudre sèche reconstituable dans le LCRa.
